(19) Europäisches Patentamt
European Patent Office
Office européen des brevets

(11) Veröffentlichungsnummer: **0 239 002**
**B1**

(12) # EUROPÄISCHE PATENTSCHRIFT

(45) Veröffentlichungstag der Patentschrift: **26.09.90**

(51) Int. Cl.⁵: **G01N 33/52,** G01N 33/86, D21H 13/40, D21H 17/36

(21) Anmeldenummer: **87103993.9**

(22) Anmeldetag: **18.03.87**

(54) **Testträger und Verfahren zur Bestimmung von Gerinnungsparametern.**

(30) Priorität: **27.03.86 DE 3610429**

(43) Veröffentlichungstag der Anmeldung: **30.09.87 Patentblatt 87/40**

(45) Bekanntmachung des Hinweises auf die Patenterteilung: **26.09.90 Patentblatt 90/39**

(84) Benannte Vertragsstaaten: **AT BE CH DE ES FR GB GR IT LI LU NL SE**

(56) Entgegenhaltungen:
**EP-A- 0 045 476**
**DE-A- 2 835 935**
**FR-A- 2 249 209**
**GB-A- 1 364 755**
**US-A- 4 050 898**

(73) Patentinhaber: **BOEHRINGER MANNHEIM GMBH, Sandhofer Strasse 116, D-6800 Mannheim 31(DE)**

(72) Erfinder: **Doeding, Jürgen, Frenaystrasse 22, D-6140 Bensheim 1(DE)**
Erfinder: **Wielinger, Hans, Dr. phil., Im Langgewann 7, D-6940 Weinheim(DE)**
Erfinder: **Lerch, Rolf, Lessingstrasse 4, D-6804 Ilvesheim(DE)**

**EP 0 239 002 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen Testträger zur Bestimmung sogenannter Gerinnungsparameter, d. h. zur Analyse des Blutes im Hinblick auf die den Gerinnungsvorgang beeinflußenden Bestandteile.

Zur qualitativen oder quantitativen analytischen Bestimmung von Bestandteilen von Körperflüssigkeiten, insbesondere von Blut, werden in jüngerer Zeit zunehmend sogenannte trägergebundene Tests verwendet. Bei diesen sind Reagenzien in entsprechenden Schichten eines festen Testträgers eingebettet, der mit der Probe in Kontakt gebracht wird. Die Reaktion von Probe und Reagenzien führt zu einem nachweisbaren Signal, insbesondere einem Farbumschlag, welcher visuell oder mit Hilfe eines Gerätes, meistens reflexionsphotometrisch, ausgewertet werden kann.

Testträger sind häufig als Teststreifen ausgebildet, die im wesentlichen aus einer länglichen Tragschicht aus Kunststoffmaterial und darauf angebrachten Testfeldern bestehen. Es sind jedoch auch Testträger bekannt, die als quadratische oder rechtreckige Plättchen gestaltet sind.

In der EP-A 00 45 476 werden Mittel und Verfahren zum Abtrennen von Plasma oder Serum aus Vollblut, insbesondere für Testträger zur diagnostischen Bestimmung von Bestandteilen von Körperflüssigkeiten (sogenannte Parameter), beschrieben, die es ermöglichen, Inhaltsstoffe aus Vollblut auf einfache Weise zu bestimmen. Gemäß dieser Literaturstelle läßt sich das Plasma von den Erythrozyten abtrennen, indem man das Vollblut durch eine Schicht aus Glasfasern mit Faserdurchmessern von unter 2, 5μ laufen läßt, welche die Erythro zyten zurückhält. Vorzugsweise wird dabei das Blut auf ein Ende eines rechteckigen Glasfaservlieses aufgetragen, von wo aus das Plasma kapillar in den übrigen Bereich transportiert wird. Auf diesen mit Plasma gefüllten Vliesteil wird nach dem Plasmagewinnungsvorgang eine die für die Reaktion notwendigen Reagenzien enthaltende Matrix (Papier, saugfähige Filme etc.) gedrückt, in der die Nachweisreaktion für den nachzuweisenden Parameter über Remissionsmessungen durchgeführt wird.

Dieses einfache Plasmagewinnungs- und Plasmatransport-System ist für alle klinisch-chemisch wichtigen Parameter einsetzbar, mit Ausnahme von Parametern, die im Rahmen der Untersuchungen bei hämostasiologischen Fragestellungen zu bestimmen sind. Es ist bekannt, daß Gerinnungsanalysen prinzipiell in Kunststoffgefäßen oder Glasgefäßen, die durch einen Belag aus Silikonharz inaktiviert sind, durchzuführen sind, weil unbehandeltes Glas die Gerinnbarkeit von Blut oder Plasma beeinflußt. So verkürzt Glas durch Aktivierung die Einphasen-Gerinnungszeit nach Quick von Plasmen, deren Gerinnungsfaktoren im Normalbereich liegen. Die Einphasen-Gerinnungszeit nach Quick wird bei niederprozentigen Plasmen durch Inaktivierung von Gerinnungsfaktoren verlängert. Glas inaktiviert dabei vor allem die Faktoren V und IIa. Durch diese Inaktivierung und damit fälschlich verlängerte Gerinnungszeiten wird der diagnostische Nutzen zunichte gemacht, weil die Verhältnisse der einzelnen Gerinnungsfaktoren verschoben werden. Der in Prozent angegebene Quick-Wert umfaßt neben der Fibrinogenkonzentration die Aktivität der Faktoren II, V, VII und X. Ein Poolplasma von gesunden Spendern wird als 100 % Plasma definiert, über Verdünnung mit physikalischer Kochsalzlösung werden entsprechend niederprozentige Plasmen hergestellt. Über diese Verdünnungsreihe wird eine Bezugskurve erstellt, anhand derer die Quick-Werte für Patientenplasmen ermittelt werden.

Aber auch die Bestimmung der partiellen Thromboplastinzeit (PTT) wird durch die Inaktivierung der Faktoren XII und XI negativ beeinflußt. Der Nachweis von Antithrombin III und Heparin wird durch eine Inaktivierung von Thrombin durch Glas erheblich gestört.

Aufgabe der vorliegenden Erfindung war es daher, insbesondere als Abtrenn- und Transport-System in einem Testträger geeignete Glasfaserschichten zu schaffen, die gerinnungsneutral und dadurch auch für hämostasiologische Untersuchungen brauchbar sind. Dabei sollen sie bevorzugt ihre Abtrenn- und Transporteigenschaften behalten.

Die Aufgabe wird gelöst durch einen Testträger zur Bestimmung von Gerinnungsparametern des Blutes, dadurch gekennzeichnet, daß er eine Testschicht (4) einschließt, welche Glasfasern enthält, die mit Polyvinylalkohol oder Polyvinylalkohol/vinylacetat überzogen sind.

Ebenso wird die Aufgabe gelöst durch ein Verfahren zur Bestimmung von Gerinnungsparametern des Blutes, dadurch gekennzeichnet, daß Blut durch eine Glasfaserschicht einer Dichte von 0,1–0,5 g/cm³ geleitet wird, die Glasfasern mit einem mittleren Durchmesser von 0,2–5 μm enthält, welche mit Polyvinylalkohol oder Polyvinylalkohol/vinylacetat überzogen sind und die Erythrozyten von Plasma trennt und daß mit dem so gewonnenen Plasma die Bestimmung von Gerinnungsparametern des Blutes durchgeführt wird.

Außerdem wird die anmeldungsgemäße Aufgabe gelöst durch Verwendung von mit Polyvinylalkohol oder Polyvinylalkohol/vinylacetat überzogenen Glasfasern zur Plasmagewinnung aus Blut, wobei die Glasfasern einen mittleren Durchmesser von 0,2–5 μm besitzen und die Glasfaserschicht eine Dichte von 0,1–0,5 g/cm³ aufweist, insbesondere bei anschließender Bestimmung von Gerinnungsparametern des Blutes. Schließlich wird die Aufgabe gelöst durch die Verwendung von mit Polyvinylalkohol oder Polyvinylalkohol/vinylacetat überzogenen Glasfasern zum Transport von Plasma durch Kapillarkraft insbesondere bei anschließender Bestimmung von Gerinnungsparametern des Blutes.

Es wurde gefunden, daß man Glasoberflächen durch Zusatz von Polyvinylalkoholen chemisch so modifizieren kann, daß sie die hydrophilen Eigenschaften, die z.B. für die Saugfähigkeit von Glasfaser-Vliesen notwendig sind, beibehalten, aber den Einfluß auf die Gerinnungsfaktoren verlieren, d.h. gerinnungsneutral werden. Dies ist besonders überraschend, weil es bekannt ist, daß man Gläser durch Silikonisierung mit Silikonharzemulsionen oberflächlich belegen kann und dadurch eine Beeinflussung der Gerinnungsfaktoren ausschließt, diese Silikonisierung jedoch im speziellen Fall der Glasfasern eine so starke Hydrophobierung der Oberfläche bewirkt, daß keine Benetzung mehr stattfinden kann und somit die Fasern ihre Plasma-Transporteigenschaften und Erythrozyten-Abtrenneigenschaften völlig verlieren.

Aus GB-A 1 364 755 sind Glasfaservliese bekannt, wobei die Glasfasern durch Polyvinylalkohol als Bindemittel zusammengehalten werden. Solche Vliese dienen dort als verstärkte Kunststoffträger für gedruckte Stromkreise. Eine Verwendung im anmeldungsgemäßen Sinn wird weder erwähnt noch nahegelegt.

Polyvinylalkohol wird aus Polyvinylacetat durch Verseifung hergestellt, wobei je nach den gewünschten Eigenschaften des Produktes eine vollständige oder teilweise Verseifung durchgeführt wird. Für den erfindungsgemäßen Zusatz ist sowohl ein voll- als auch ein teil-verseiftes Produkt verwendbar. Polyvinylalkohole, die im Handel in großer Menge angeboten werden, unterscheiden sich insbesondere durch ihr durchschnittliches Molekulargewicht, welches normalerweise zwischen etwa 10.000 und 100.000 liegt und in einigen Sonderfällen auch noch wesentlich höhere Werte erreichen kann, sowie durch den Restgehalt an Acetylgruppen. Die niedermolekularen Verbindungen, die ca. 5-15%, insbesondere etwa 10 % Acetylgruppen enthalten, sind in Wasser am leichtesten löslich, hochmolekulare und/.oder stärker Acetylgruppen-haltige Produkte sind dagegen in Wasser weniger löslich. Von Einfluß auf die Löslichkeit ist ferner die Wechselwirkung der Polyvinylalkoholketten untereinander. Durch eine parallele Lagerung der Polymerketten in bestimmten Bereichen entstehen "kristalline Bezirke", wobei die Orientierungstendenz umso größer ist, je regelmäßiger die Ketten aufgebaut sind und je geringer der Anteil der Acetylgruppen ist, die einer Orientierung am stärksten entgegenwirken. Bei einem Verseifungsgrad von 97-100 Mol-%, d. h. bei einem Acetylierungsgrad von 3-0 Mol-% nimmt deshalb die "Kristallinität" besonders stark zu, wodurch umgekehrt die Kaltwasserlöslichkeit stark abnimmt.

Die Wasserlöslichkeit kann weiterhin durch Nachbehandlung mit Aldehyden (Acetalisierung) oder andere chemische Umwandlung der Alkoholgruppen verringert werden. Erfindungsgemäß brauchbar sind insbesondere die Polyvinylalkohole mit einer geringen Kaltwasserlöslichkeit, aber guten Löslichkeit in warmem Wasser. Die Produkte sollen bei 20°C in Wasser nur langsam oder überhaupt nicht gelöst werden, jedoch bei Temperaturen von 50-100°C, insbesondere bei Temperaturen von über 60°C sich in Wasser leicht lösen.

Die Herstellung der erfindungsgemäßen Glasfaservliese kann dadurch erfolgen, daß ein entsprechendes Glasfaservlies nachträglich mit einer Lösung des Polyvinylalkohols in Wasser oder einem geeigneten organischen Lösungsmittel behandelt und anschließend getrocknet wird, vorzugsweise bei Temperaturen von über 60 °C, ganz besonders bei 90°C–140°C, oder indem man bei der Herstellung der Glasfaservliese Polyvinylalkohol zusetzt. Bekanntlich werden Glasfaservliese so hergestellt, daß man die trockenen und verfilzten Glasfasern, die einen mittleren Durchmesser von 0,1–20 µm und eine Länge von 0,1–5 mm aufweisen, in einem sehr großen Überschuß von Wasser suspendiert, dadurch vereinzelt und diese "Bütte" analog den in der Papierherstellung üblichen Verfahren und mit Hilfe der dort üblichen Maschinen zu dünnen Schichten formt und trocknet. Der Bütte zugesetzte Polyvinylalkoholpulver oder -fasern verteilen sich bei der Aufschlämmung der Glasfasern gleichmäßig in der Masse und werden beim anschließenden Herstellen des Vlieses soweit gelöst bzw. aufgeschmolzen, daß sie anschließend an die Trocknung des Vlieses einen gleichmäßigen Überzug auf den Glasfasern bilden. Dieser Überzug bewirkt, daß die Glasfasern gerinnungsneutral werden, andererseits aber noch soweit hydrophile Eigenschaften besitzen, daß die Saugfähigkeit und der Transport von Wasser bzw. wässrigen Lösungen durch diese Vliese nicht beeinträchtigt wird.

Besonders bevorzugt werden in die Bütte silanisierte Glasfasern gegeben. Geeignete derartige Fasern sind in der Deutschen Patentanmeldung 3 523 969 beschrieben, auf welche hier vollinhaltlicher Bezug genommen wird. Diese deutsche Patentanmeldung betrifft gerinnungsneutrale, hydrophile Glasfasern, an deren Oberfläche Silane gebunden sind sowie Verfahren zu ihrer Herstellung durch Umsetzung von Glasfasern mit einem Silan der Formel I

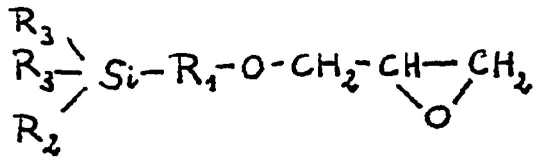 (I)

worin

$R_1$ eine Alkylengruppe mit 2–6 C-Atomen

$R_2$ eine niedere Alkoxygruppe mit 1–6 C-Atomen und

$R_3$ eine niedere Alkyl- oder niedere Alkoxygruppe mit 1–6 CAtomen

bedeutet. Solche silanisierten Glasfasern werden dann zusätzlich gemäß der vorliegenden Erfindung mit Polyvinylalkohol oder Polyvinylalkohol/vinylacetat überzogen. Dadurch ergeben sich Glasfaserschichten, die in besonderem Maße gerinnungsneutral sind und sich durch besonders gute Festigkeit auszeichnen.

Da der Polyvinylalkohol die Glasfasern relativ gleichmäßig überzieht, wenn er erfindungsgemäß aufgebracht wird, reichen bereits relativ kleine Mengen, insbesondere etwa 0,5-20 %, vorzugsweise 1-5 %, aus, um die Fasern gerinnungsneutral zu machen. Anteile von über 20 % wären zwar für den beabsichtigten Effekt nicht schädlich, sind aus wirtschaftlichen Gründen jedoch nicht sinnvoll.

Soweit die Glasfaservliese nicht nur zum Transport von Serum und Plasma, sondern auch zur Abtrennung der Erythrozyten aus Plasma gemäß EP-A 00 45 476 verwendet werden sollen, sollten die Glasfasern einen mittleren Durchmesser von 0,2-5µm, vorzugsweise 0,5-2,5µm, besitzen und die Vliese eine Dichte von 0,1-0,5 g/cm$^3$ aufweisen. Soweit es lediglich auf die Gerinnungsneutralität und die Transporteigenschaften ankommt, können auch Glasfasern größerer Dicke und Vliese anderer Dichte eingesetzt werden. Auch der Polyvinylalkoholgehalt des Vlieses kann bei solchen Vliesen über den sonst vorteilhaften Gehalt von 20 % hinaus gesteigert sein.

Die erfindungsgemäßen Glasfasern werden nach herkömmlichen Verfahren zu Vliesen verlegt, die, wie in den u.a. Beispielen beschrieben wird, in Mitteln zur Bestimmung von Gerinnungsparametern eingesetzt werden können. Sie können jedoch auch in Form einer kurzen Säule zur Plasmagewinnung gemäß EP-A 0045 476 verwendet werden, worauf das Plasma dann in herkömmlicher Weise in Gerinnungstesten eingesetzt werden kann.

Weitere Ausgestaltungsmöglichkeiten der erfindungsgemäßen Testträger zur Bestimmung von Gerinnungsparametern können in Analogie zu den in der EP-A 00 45 476 beschriebenen Mitteln, auf die hiermit Bezug genommen wird, hergestellt werden. Es ist selbstverständlich, daß in solchen Mitteln nicht nur die erfindungsgemäßen Glasfaserschichten, sondern auch alle übrigen mit dem Plasma in Kontakt kommenden Teile aus gerinnungsneutralen Materialien bestehen müssen, wobei insbesondere entsprechende, dem Fachmann bekannte Kunststoffe eingesetzt werden.

Die Erfindung wird im folgenden anhand der Figuren und mehrerer Beispiele näher erläutert.

Es zeigen:

Figur 1 eine schematische Seitenansicht eines erfindungsgemäßen Testträgers

Figur 2 eine Remissionskurve, die unter Verwendung eines Testträgers gemäß Figur 1 hergestellt wurde.

Der in Figur 1 dargestellte Testträger 1 hat die prinzipielle Form eines konventionellen Teststreifens. Auf einer Tragschicht 2 aus einer Kunststoffolie (z. B. Polystyrol) ist mit einem Schmelzkleberstreifen 3 eine Glasfaserschicht 4 und ein die Glasfaserschicht 4 teilweise überlappendes Abdecknetz 3a befestigt.

Auf der anderen Seite wird die Glasfaserschicht 4 von einer Oxidationsmittelschicht 5 und einer Reaktionsschicht 6 überlappt, welche klappenartig mit einem weiteren Schmelzkleberstreifen 7 an der Tragschicht 2 befestigt sind.

Zur Durchführung einer Analyse wird ein Blutstropfen auf das Abdecknetz 3a aufgegeben und über die Glasfaserschicht 4 in den Bereich der Oxidationsmittelschicht 5 und der Reaktionsschicht 6 transportiert. Dabei findet eine Abtrennung des Plasmas statt, wie dies in der Europäischen Patentanmeldung 45476 beschrieben ist. Um die Reaktion mit den auf den Schichten 5 und 6 enthaltenen Reagenzien einzuleiten, werden diese nach unten gedrückt, so daß die Probenflüssigkeit in diese Schichten eindringt und die entsprechenden Reaktionen ablaufen.

Nähere Einzelheiten über die bei einem Gerinnungstest der vorliegenden Art ablaufenden chemischen Reaktionen sind der Europäischen Patentanmeldung mit der Veröffentlichungsnummer 182373 und den folgenden Beispielen zu entnehmen.

Beispiel 1:

Herstellung eines erfindungsgemäßen Glasfaservlieses

1 kg Glasfasern werden mit 420 Litern Wasser, das leicht sauer (pH 3,0) eingestellt ist, zur Vereinzelung der Fasern im Holländer aufgeschlagen. Im Vorratsbehälter einer Papiermaschine (Schrägsiebmaschine Void, Heidenheim) wird auf eine Stoffdichte von 0,3 % verdünnt und dieser Verdünnung 50 g Polyvinylalkoholfasern (Kuralon VPB 105-2 der Firma Kuraray) zugesetzt und vermischt. Im Stoffauflauf der Papiermaschine wird die Suspension mit Wasser aus dem Kreislauf auf 0,05 % verdünnt und so auf das Sieb gebracht. Hier erfolgt die Blattbildung. Das Blatt wird auf dem Trockenzylinder bei 110-120°C getrocknet. Je nach Einstellung der Maschine entstehen Vliese mit Flächengewichten von 20- 100 g/m$^2$ und Dicken von 0,2-1 mm.

Beispiel 2:

Prüfen der Glasfaservliese auf Gerinnungsneutralität

Von den gemäß Beispiel 1 hergestellten Glasfaservliesen werden Proben von je 40 mg mit je 300 µl Poolplasma von gesunden Spendern als auch von mit Antikoagulantien behandelten Patienten zusammengebracht und 1 Minute bei 37°C inkubiert. Danach werden die Plasmen über Zentrifugation abgetrennt. Die Plasmen werden vor und nach der Behandlung mit den Glasfaservliesen auf die Einphasengerinnungszeit nach Quick untersucht. Dabei kann der Clotting-Test verwendet werden: Starten der Gerinnungskaskade Calciumchlorid und Thromboplastin und häkeln der Probe, wobei die Zeit bis zur Bildung der Fibrinfäden gemessen wird. Ebenso kann ein photometrischer Test zur Bestimmung der Quick-Zeit herangezogen werden (Becker, U. et al.: Neue Aspekte der Gerinnungsdiagnostik, F.K. Schattauer-Verlag, Stuttgart - New York, 1984, Seite 17 bis 30).

Beispiel 3:

Bestimmung der Einphasengerinnungszeit nach Quick

a) Herstellung des Testträgers

Es wird ein Testträger gemäß Figur 1 hergestellt. Die Glasfaserschicht 4 ist ein Vlies mit einem Flächengewicht von 50 - 60 g/m² und eine Dicke von 0,5 mm. Es ist in Längsrichtung des Testträgers etwa 15 mm lang. Das Abdecknetz 3a hat eine Fadendicke von 140 µm und eine Maschenweite von 250 µm.

Die Reaktionsschicht 6 besteht aus einer Polycarbonatfolie von 200 µm Stärke, auf welche ein Reagenzfilm in einer Naßfilmdicke von 110 µm aufgerakelt und bei 45°C getrocknet wird. Die filmbildende Masse wird wie folgt hergestellt:

In 1 Liter Wasser werden gelöst: 10 g eines linear vernetzten Polyacrylamides (Rohagit 700 der Firma Röhm, Darmstadt); 4-(2-Hydroxyethyl)-1-piperazin-ethansulfonsäure 100 mmol; Tos-Gly-Pro-Arg-p-phenylendiamin 1 mmol; N-(4-fluorphenyl)-N-methylaminomethan-phosphonsäure 15 mmol; Kaninchenhirnthromboplastin 4,9 g. Diese Lösung wird mit Natronlauge auf einen pH von 7,5 eingestellt.

Die Oxidationsmittelschicht 5 besteht aus einem entsprechend imprägnierten Nylonnetz (Fadenstärke ca. 40 µm, Maschenweite ca. 60 µm, Type NY 20 HC Super der Firma Züricher Beuteltuchfabrik, Schweiz). Das Netz wird mit einer wässrigen Lösung von 50 mmol $K_3[Fe(CN)_6]$/1 und 50 mmol Calciumchlorid/1 imprägniert.

b) Vergleich zwischen erfindungsgemäßen Testträgern und solchen, bei denen normale Glasfaservliese eingesetzt werden.

Auf das Nylonnetz 3a, mit dem das Glasfaservlies 4 fixiert ist, werden je 35 µl Citratplasma aus einer Verdünnungsreihe von 100 %, 50 %, 33 %, 25 % kund 12,5 % in physiologischer Kochsalzlösung pipettiert. Die Testträger werden dann auf 37° C temperiert und mit einem Remissionsphotometer die Farbbildung nach der Zeit verfolgt. Dabei ist zu beobachten, daß mit den erfindungsgemäßen Glasfaservliesen wesentlich höhere Signale erhalten werden als mit den unbehandleten Glasfaservliesen (siehe Figur 2). Als Quick-Zeit wird die Zeit genommen, innerhalb derer eine Remissionsabnahme von 10 % Remission durchlaufen wird. Die Unterschiede werden in der folgenden Tabelle in Zahlen dargestellt:

| Plasma | Test mit erfindungsgemäßen Glasfaservliesen<br>Zeiten in Sekunden für eine eine Remissionsabnahme um 10 % | Test mit nichtbeschichtet Glasfaservliesen<br>Zeiten in Sekunden für eine Remissionsabnahme um 10 % |
|---|---|---|
| 100 % | 44,7 | 46,2 |
| 50 % | 51,5 | 78,8 |
| 33 % | 58,8 | 121,7 |
| 25 % | 66,0 | 160,2 |
| 12,5 % | 99,0 | nicht mehr meßbar |

## Patentansprüche

1. Testträger zur Bestimmung von Gerinnungsparametern des Blutes, dadurch gekennzeichnet, daß er eine Testschicht (4) einschließt, welche Glasfasern enthält, die mit Polyvinylalkohol oder Polyvinylalkohol/vinylacetat überzogen sind.
2. Testträger nach Anspruch 1, dadurch gekennzeichnet, daß die Testschicht (4) 0,5–20%, vorzugsweise 1–5%, Polyvinylalkohol enthält.
3. Testträger nach Anspruch 1, dadurch gekennzeichnet, daß die Glasfasern einen mittleren Durchmesser von 0,2–5 µm, vorzugsweise 0,5–2,5 µm besitzen und die Testschicht eine Dichte von 0,1–0,5 g/cm³ aufweist.
4. Testträger nach Anspruch 1, dadurch gekennzeichnet, daß die Glasfasern silanisiert sind.
5. Testträger nach Anspruch 1, dadurch gekennzeichnet, daß die Testschicht als Vlies ausgebildet ist.
6. Verfahren zur Bestimmung von Gerinnungsparametern des Blutes, dadurch gekennzeichnet, daß Blut durch eine Glasfaserschicht einer Dichte von 0,1–0,5 g/cm³ geleitet wird, die Glasfasern mit einem mittleren Durchmesser von 0,2–5 µm enthält, welche mit Polyvinylalkohol oder Polyvinylalkohol/vinylacetat überzogen sind und die Erythrozyten von Plasma trennt und daß mit dem so gewonnenen Plasma die Bestimmung von Gerinnungsparametern des Blutes durchgeführt wird.
7. Verfahren nach Anspruch 6, dadurch gekennzeichnet, daß es mittels eines Testträgers gemäß einem der Ansprüche 1–5 durchgeführt wird.
8. Verwendung von mit Polyvinylalkohol oder Polyvinylalkohol/vinylacetat überzogenen Glasfasern zur Plasmagewinnung aus Blut, wobei die Glasfasern einen mittleren Durchmesser von 0,2 bis 5 µm besitzen und die Glasfaserschicht eine Dichte von 0,1–0,5 g/cm³ aufweist, insbesondere bei anschließender Bestimmung von Gerinnungsparametern des Blutes.
9. Verwendung von mit Polyvinylalkohol oder Polyvinylalkohol/vinylacetat überzogenen Glasfasern zum Transport von Plasma durch Kapillarkraft insbesondere bei anschließender Bestimmung von Gerinnungsparametern des Blutes.

## Claims

1. Test carrier for the determination of coagulation parameters of blood, characterised in that it includes a test layer (4) which contains glass fibres which are coated with polyvinyl alcohol or polyvinyl alcohol/vinyl acetate.
2. Test carrier according to claim 1, characterised in that the test layer (4) contains 0.5–20%, preferably 1–5%, polyvinyl alcohol.
3. Test carrier according to claim 1, characterised in that the glass fibres possess an average diameter of 0.2–5 µm, preferably 0.5–2.5 µm and the test layer has a density of 0.1–0.5 g/cm³.
4. Test carrier according to claim 1, characterised in that the glass fibres are silanised.
5. Test carrier according to claim 1, characterised in that the test layer is formed as fleece.
6. Process for the determination of coagulation parameters of blood, characterised in that blood is passed through a glass fibre layer with a density of 0.1–0.5 g/cm³, which contains glass fibres with an average diameter of 0.2–5 µm which are coated with polyvinyl alcohol or polyvinyl alcohol/vinyl acetate

and the erythrocytes are separated from plasma and that the determination of coagulation parameters of the blood is carried out with the so-obtained plasma.

7. Process according to claim 6, characterised in that it is carried out by means of a test carrier according to one of claims 1–5.

8. Use of glass fibres coated with polyvinyl alcohol or polyvinyl alcohol/vinyl acetate for obtaining plasma from blood, whereby the glass fibres possess an average diameter of 0.2 to 5 µm and the glass fibre layer has a density of 0.1–0.5 g/cm³, especially in the case of subsequent determination of coagulation parameters of blood.

9. Use of glass fibres coated with polyvinyl alcohol or polyvinyl alcohol/vinyl acetate for the transport of plasma by capillary force, especially in the case of subsequent determination of coagulation parameters of blood.

**Revendications**

1. Support de test pour la détermination des paramètres de coagulation du sang, caractérisé en ce qu'il comprend une couche de test (4) qui contient des fibres de verre, qui sont enrobées d'alcool polyvinylique ou d'alcool polyvinylique/acétate de vinyle.

2. Support de test selon la revendication 1, caractérisé en ce que la couche de test (4) contient 0,5–20% de préférence 1–5% d'alcool polyvinylique.

3. Support de test selon la revendication 1, caractérisé en ce que les fibres de verre présentent un diamètre moyen de 0,2–5 µm, de préférence 0,5–2,5 µm, et la couche de test présente une densité de 0,1–0,5 g/cm³.

4. Support de test selon la revendication 1, caractérisé en ce que les fibres de verre sont silanisées.

5. Support de test selon la revendication 1, caractérisé en ce que la couche de test est constituée sous forme de nappe.

6. Procédé pour la détermination des paramètres de coagulation du sang, caractérisé en ce que le sang est conduit à travers und couche de fibres de verre ayant une densité de 0,1–0,5 g/cm³, contenant des fibres de verres ayant une diamètre moyen de 0,2–5 µm, qui sont enrobées d'alcool polyvinylique ou d'alcool polyvinylique/acétate de vinyle et que les érythrocytes sont séparés du plasma et que la détermination des paramètres de coagulation du sang est réalisée au moyen du plasma ainsi obtenu.

7. Procédé selon la revendication 6, caractérisé en ce qu'il est effectué au moyen d'un support de test selon l'une quelconque des revendication 1–5.

8. Utilisation de fibres de verre enrobées d'alcool polyvinylique ou d'alcool polyvinylique/acétate de vinyle, pour l'obtention de plasma a partir du sang, les fibres des verre présentant un diamètre moyen de 0,2 à 5 µm et la couche de fibres de verre présentant une densité de 0,1–0,5 g/cm³, en particulier dans la détermination subséquente des paramètres de coagulation du sang.

9. Utilisation de fibres de verre enrobées d'alcool polyvinylique ou d'alcool polyvinylique/acétate de vinyle, pour le transport du plasma par capillarité, en particulier dans la détermination subséquente des paramètres de coagulation du sang.

FIG. 1

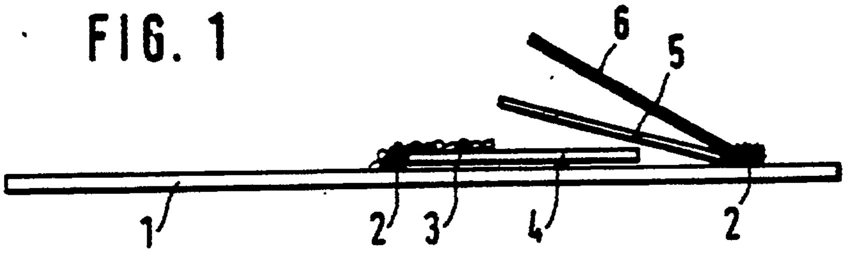


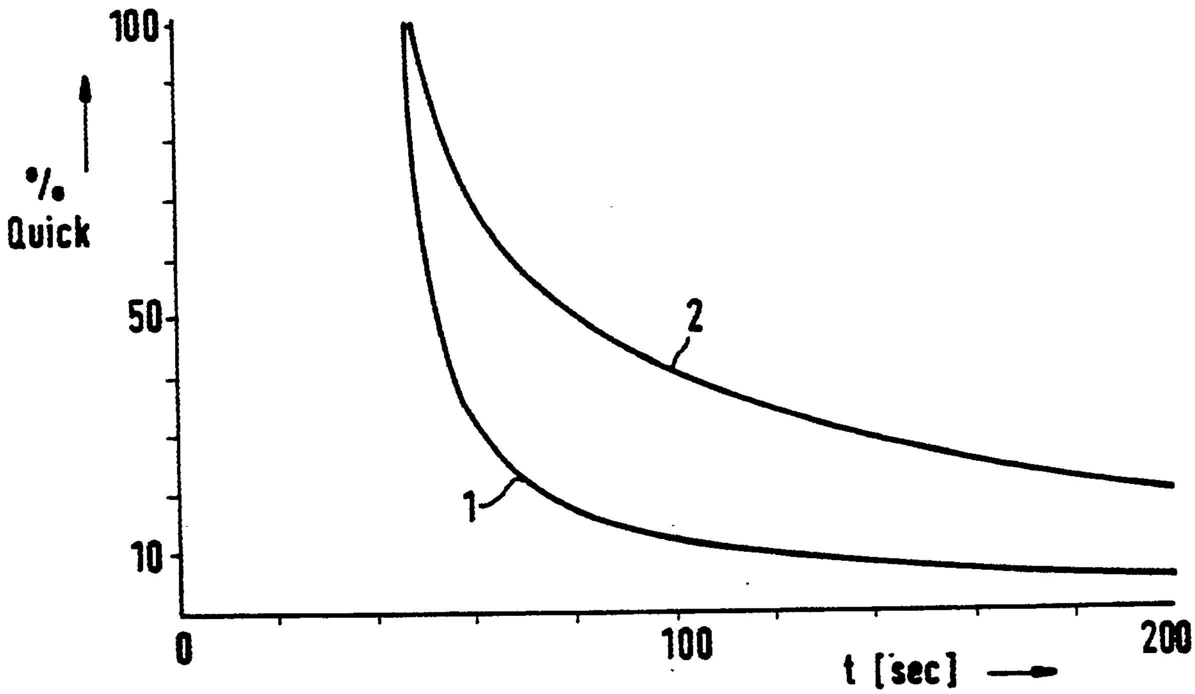


FIG.2